Europäisches Patentamt

(19) European Patent Office (11) Veröffentlichungsnummer: **0 155 341**
**B1**
Office européen des brevets

(12) # EUROPÄISCHE PATENTSCHRIFT

(45) Veröffentlichungstag der Patentschrift: (51) Int. Cl.⁴: **B 01 J 8/02**, C 07 C 29/15
09.03.88

(21) Anmeldenummer: **84105393.7**

(22) Anmeldetag: **12.05.84**

(54) Stehender Reaktor zur Erzeugung von Methanol.

(30) Priorität: **06.09.83 DE 3332049**
**08.12.83 DE 3344423**

(43) Veröffentlichungstag der Anmeldung:
**25.09.85 Patentblatt 85/39**

(45) Bekanntmachung des Hinweises auf die Patenterteilung:
**09.03.88 Patentblatt 88/10**

(84) Benannte Vertragsstaaten:
**AT DE FR GB IT NL**

(56) Entgegenhaltungen:
**DE - A - 1 501 640**
**DE - A - 2 549 398**
**DE - A - 2 848 014**
**DE - A - 3 007 203**
**DE - A - 3 022 815**
**DE - A - 3 117 077**
**DE - C - 914 131**
**US - A - 4 298 589**

(73) Patentinhaber: **MAN GUTEHOFFNUNGSHÜTTE GMBH,**
**Bahnhofstrasse, 66 Postfach 11 02 40,**
**D-4200 Oberhausen 11 (DE)**

(72) Erfinder: **Vollhardt, Frohmut, Nettelbeckstrasse 2,**
**D-4200 Oberhausen 11 (DE)**

**Beschreibung**

Die Erfindung betrifft einen stehenden Reaktor zur Herstellung von Methanol, mit einem zylindrischen Gehäuse mit das Katalysatorbett im Inneren des Gehäuses durchsetzenden, parallel zur Reaktorlängsachse verlaufenden Austauscherrohren sowie einem das Katalysatorbett tragenden, gasdurchlässigen Boden oder Netz im unteren Reaktorabschnitt, bei dem das Gehäuse durch eine obere Haube abgeschlossen ist, das Katalysatorbett von einem zylindrischen Mantel innerhalb des Gehäuses umschlossen ist, der bei abgehobener Haube mit dem Katalysatorbett in Längsrichtung des Reaktors aus dessen Gehäuse heraushebbar ist.

Aus der DE-A-2 848 014 sowie der DE-A-2 549 398 sind stehende Reaktoren zur Herstellung von Methanol nach dem Oberbegriff des Anspruchs 1 bekannt.

In der DE-A-3 007 203 wird ein Reaktor zur Durchführung einer katalytischen Methanolsynthese beschrieben mit einem zylindrischen Gehäuse, das die auf einem Lochblech ruhende Katalysatorschüttung aufnimmt und mit in der Schüttung verlaufenden Tauscherrohren zur Führung eines Kühlfluids versehen ist. Die Rohre sind als Flossenrohre ausgebildet und verlaufen geradlinig in der Schüttung sowie parallel zur Gehäuseachse. Die freien Enden der Tauscherrohre münden in einen oberen oder unteren Rohrboden.

Ein Wärmetauscher nach der DE-A-1 501 640 dient insbesondere zum Kühlen von frischen Spaltgasen und/oder Synthesegas und weist einen Mantel aus Flossenrohren auf, und zwar sind die Austauscherrohre derart mit Flossen versehen, dass geschlossene geometrische Räume entstehen.

Bei dem aus der DE-C-914 131 bekannten Kontaktofen befindet sich der Reaktor in einem zylindrischen Gehäuse. Der Reaktor kann nicht aus dem Gehäuse herausgehoben werden, sondern es können lediglich jeweils Rohreinheiten herausgehoben werden.

Den bekannten Methanol-Reaktoren mit den Merkmalen des Gattungsbegriffes des Hauptanspruches gegenüber besteht die Aufgabe der Erfindung darin, einen Reaktor zu schaffen, bei dem sowohl der Austausch der Katalysatorfüllung wie auch das Überwachen und Auswechseln der Wärmetauscherrohre in dem Katalysatorbett und das Ersetzen des Katalysatorbodens oder -netzes leicht, bei konstruktiv einfachem Reaktoraufbau, erfolgen kann. Trotz dieser vorstehend genannten Forderungen sollen die Strömungsverhältnisse in dem Katalysatorbett zur Erzielung einer langen Lebensdauer der Tauscherrohre gegenüber bekannten Rohren für den gleichen Zweck verbessert werden.

Zur Lösung dieser Aufgabe sieht die Erfindung die Merkmale des kennzeichnenden Teils des Hauptanspruches vor. Die Merkmale der Unteransprüche dienen der Weiterentwicklung und Verbesserung der Merkmale des Hauptanspruches.

Der Vorteil des erfindungsgemässen Reaktors ist darin zu sehen, dass der zylindrische Mantel, der aus Flossenrohren besteht, mit der Katalysatorfüllung aus dem Gehäuse mit seinem Druckmantel und dem Isoliermantel geschlossen herausgenommen werden kann, wonach es nur des ganzen oder des partiellen Lösens des Katalysatorbodens oder -netzes bedarf, um den Katalysator frei nach unten aus dem zylindrischen Mantel entweichen zu lassen. Wird der Katalysatorboden oder -netz wieder geschlossen, so kann die Füllung der Katalysatormasse in den zylindrischen Flossenrohrmantel leicht erfolgen.

Gleiches gilt auch hinsichtlich des Auswechselns der Tauscherrohre. Diese brauchen nicht aus dem Gehäuse des Katalysators entfernt zu werden, vielmehr können sie leicht zugänglich einzeln aus dem oben im ganzen Querschnitt offenen zylindrischen Mantel ausgebaut bzw. in diesen eingebaut werden. Die Strömungsverhältnisse innerhalb des Katalysatorbettes werden dadurch verbessert, dass zwischen den Rohren gleichmässige geometrische Strömungsräume als Aufnahmeräume für die Katalysatormasse gebildet werden, die in sich abgeschlossen sind und ein Vagabundieren des Gases in benachbarte Räume verhindern. Hierdurch wird eine gleichmässige Beaufschlagung der Austauscherrohre erzielt.

Der Vorteil der Flossenaustauscherrohre mit in die Strömungsräume ragenden Stiften besteht darin, dass die Katalysatormasse der Stifte eine Entlastung von dem Gewicht der Katalysatormasse oberhalb der Stifte bekommt und hierdurch eine Auflockerung der Katalysatormasse gegeben ist, durch die die Strömungsverhältnisse innerhalb des Katalysatorbettes verbessert werden. Desweiteren dienen die Stifte an den Rohren zur Erhöhung des Wärmeüberganges von der Katalysatormasse auf die Rohre und damit auf das Austauschermedium.

An sich ist es in der Technik bekannt, Wände zu bestiften, so z. B. die Wände von Schmelzkammern, um die Stampfmasse oder Auskleidung der Schmelzkammer zu stützen und zu halten. Hier liegt somit eine andere Aufgabe und Wirkung als beim Erfindungsgegenstand vor.

Der erfindungsgemässe Reaktor eignet sich nicht nur für die Erzeugung von Methanol, sondern in ihm können auch andere katalytische Umsetzungen durchgeführt werden.

Auf der Zeichnung sind Ausführungsbeispiele des Reaktors nach der Erfindung dargestellt, und zwar zeigt

Fig. 1 eine erste Ausführungsform eines Reaktors im Längsschnitt mit seinem Reaktorbett,

Fig. 2 eine weitere Ausführungsform eines Reaktors, bei welchem der Innenraum des zylindrischen Mantels zwei Reaktorbetten aufnimmt,

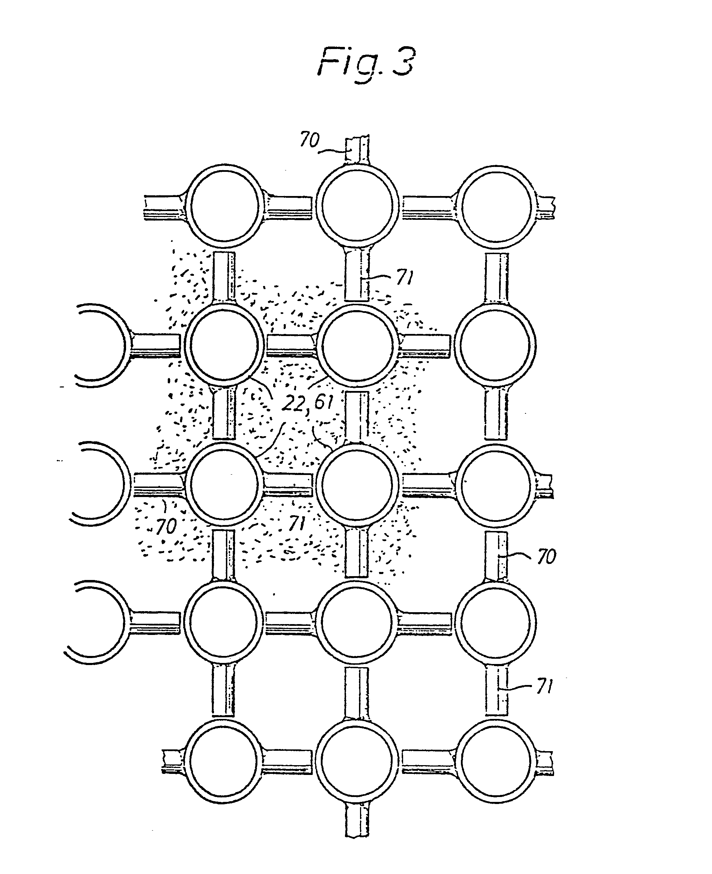

Fig. 3 einen horizontalen Querschnitt durch das Katalysatorbett bzw. die Katalysatorbetten der beiden Ausführungsformen nach Fig. 1 und 2

Fig. 4 einen weiteren Horizontalschnitt durch den Reaktor und

Fig. 5 einen Schnitt nach der Linie V − V der Fig. 4.

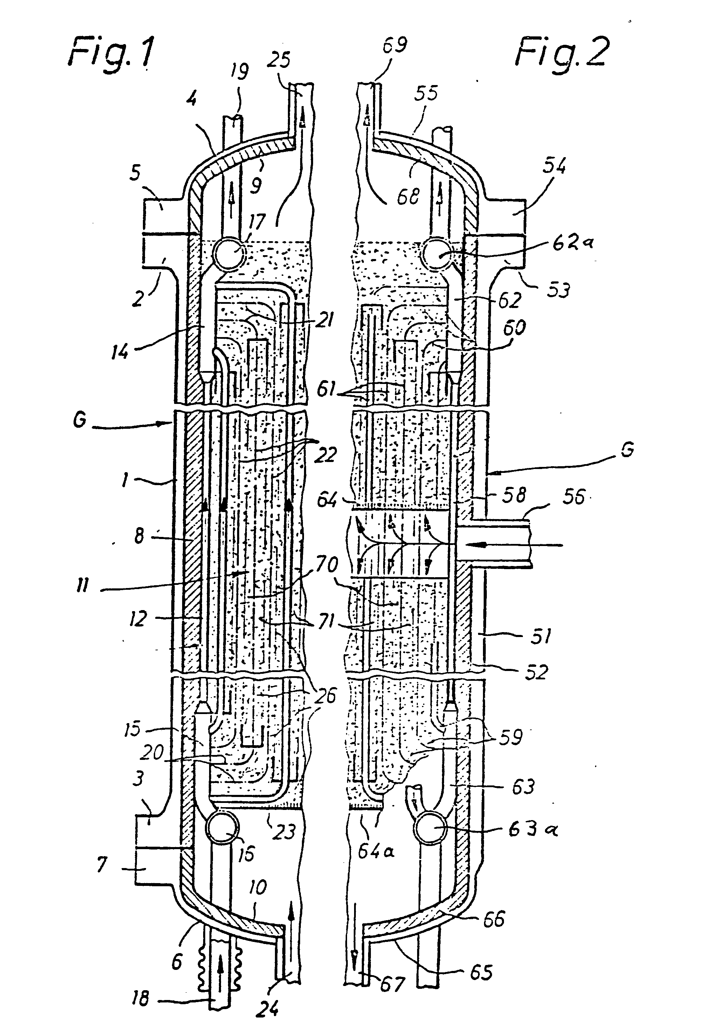

Bei der Ausführungsform des stehenden Reaktors nach Fig. 1, weist dessen Gehäuse G einen äusseren, zylindrischen Druckmantel 1 auf, der sich von dem oberen Abschnitt zum unteren Abschnitt des Reaktors erstreckt und an seinem oberen und unteren Rand je einen Flansch 2, 3 trägt. Den Abschluss des Gehäuses G nach oben bildet eine Haube 4, die mittels ihres Flansches 5 mit dem Flansch 2 des zylindrischen Druckmantels verbunden ist, während der Boden 6 des Gehäuses G mit seinem Flansch 7 mit dem Flansch 3 verbunden ist.

Der Druckmantel 1 ist mit einer Isolierlage 8 ausgekleidet, die von der Trennfuge zwischen den Flanschen 2, 5 zur Trennfuge der Flanschen 3, 7 reicht. Die Haube 4 und der Boden 6 sind ihrerseits mit Isolierlagen 9, 10 ausgekleidet. Die Ausbildung des Druckmantels 1 und der Haube 4 bzw. des Bodens 6 mit ihren Auskleidungen ist so getroffen, dass die Haube 4 von dem Druckmantel 1 ohne Schwierigkeiten entfernt werden kann. Das gleiche gilt, falls dies notwendig ist, für den Boden 6.

Die Isolierlage 8, die den zylindrischen Druckmantel 1 auskleidet, nimmt den mittleren Abschnitt eines Wärmetauschers 11 auf. Dieser weist einen, an seinem Umfang geschlossenen, aus Flossenrohren 12 gebildeten zylindrischen Mantel auf. Das obere und untere Ende der Flossenrohre 12 mündet in einen verstärkten Rohrabschnitt 14, 15, wobei diese Rohrabschnitte in eine untere Verteilerringleitung 16 und eine obere Sammelringleitung 17 münden. Das Kühlmedium für den Wärmetauscher 11 wird bei der Ausführungsform nach Fig. 1 über eine oder mehrere, den Boden 6 und dessen Isolierlage 10 durchsetzende Rohre 18 zugeführt, während das erwärmte Kühlmedium aus der Sammelringleitung 17 über ein oder mehrere Rohre 19 abgezogen wird.

In die verstärkten Rohrabschnitte 14, 15 der Flossenrohre 12 des zylindrischen Wärmeaustauschermantels münden die in horizontale Ebenen abgebogenen Enden 20, 21 der senkrechten und parallel zur Längsachse des Reaktors verlaufenden Tauscherrohre 22 des Wärmetauschers 11, so dass ein Rohrbündel an Tauscherrohren gebildet wird.

Auf Höhe der Ringleitung 16 oder aber, wie in Fig. 1 dargestellt ist, etwas oberhalb dieser Ringleitung erstreckt sich über den inneren, freien Querschnitt dieser Ringleitung oder zwischen den Rohrabschnitten 15 ein Katalysatornetz oder -boden 23, auf dem die Katalysatormasse aufliegt, die zwischen den Rohren 22 Platz findet und sich von dem Katalysatornetz oder -boden bis etwa auf Höhe der Trennfuge zwischen den Flanschen zwischen den Flanschen 2 und 5 erstreckt.

Der Boden 6 mit seiner Isolierlage 10 weist die Rohgaszuführungsleitung 24 auf, während die Haube 4 mit ihrer Auskleidung 9 die Methanolgasabzugsleitung 25 besitzt. Das Rohgas durchströmt das Katalysatorbett 26 zwischen den Rohren 22 und den abgebogenen Enden 20, 21 der Rohre 22, um nach der Reaktion mit Hilfe des Katalysators als Methanolgas durch das Rohr 25 abgeführt zu werden.

Der an seinem Umfang geschlossene zylindrische Mantel aus Flossenrohren 12 liegt eng an der Isolierlage 8 des Reaktors an. Bei Lösen der Haube 4 mit ihrer Isolierlage 9 kann der Wärmetauscher 11 mit seinem Mantel und der Katalysatormasse nach oben aus dem Reaktor entfernt werden. Daraufhin kann durch partielles oder gänzliches Lösen des Katalysatornetzes oder -bodens 23 die Katalysatormasse aus dem Mantel abgezogen und ausgetauscht werden. Die Rohre des Wärmetauschers 11 können leicht ausserhalb des Gehäuses nach oben oder unten aus- bzw. in diesen eingebaut werden.

Bei der Ausführungsform nach Fig. 2 ist wiederum ein äusserer Druckmantel 51 vorgesehen, der von einer Isolierlage 52 ausgekleidet ist. Der Mantel 51 besitzt einen Flansch 53, mit dem der Flansch 54 einer Haube 55 verbindbar ist. Die Zuführung des Rohgases erfolgt durch eine Leitung 56 etwa auf mittlerer Höhe des Reaktors, die den Druckmantel 51, wie auch dessen Isolierlage 52 durchsetzt. Die Isolierlage umschliesst in gleicher Weise wie bei der Ausführungsform nach Fig. 1 einen Wärmetauscher. Dieser weist einen äusseren Flossenrohrmantel aus Flossenrohren 58 auf, die auf Höhe der Leitung 56 ausgespart sind, so dass das Rohgas in das Innere des Mantels einzutreten vermag. Auch sind in Fig. 1 mit ihren Enden 59, 60 abgebogene, zu einem Rohrbündel zusammengefasste Tauscherrohre 61 vorgesehen, die in verstärkte Abschnitte 62, 63 der Flossenrohre 58 münden, die ihrerseits in einer Ringleitung 62a, 63a enden, denen das Kühlmedium zu- bzw. von ihnen abgeführt wird.

Oberhalb der Zuführungsleitung 56 erstreckt sich, von den Rohren 61 durchsetzt, ein Katalysatornetz 64. Auf Höhe oder oberhalb der Ringleitung 63a befindet sich ein zweites Katalysatornetz 64a. Bei dem in Fig. 2 dargestellten Ausführungsbeispiel ist der Boden 65 mit seiner Isolierlage 66 einteilig mit dem Mantel 51 und seiner Isolierlage 52 verbunden und wird von einer Methanolgas abführenden Leitung 67 ebenso durchsetzt, wie die Haube 55 mit ihrer Isolierlage 68 von einer Leitung 69 durchsetzt wird.

Bei der Ausführungsform nach Fig. 1 und 2 besitzen die Rohre 22 bzw. 61 paarweise gegenüberliegende Flossen 70, 71 und sind derart angeordnet, dass die Flossen der Rohre einen geschlossenen Strömungsraum 72 für das die Katalysatormasse in dem Raum 72 durchströmende Gas entstehen lassen. Ein Vagabundieren des Gases in benachbarte Räume und damit eine ungleichmässige Beaufschlagung der Rohre 22, 61 wird hierdurch weitgehend vermieden. Beim partiellen Öffnen der Katalysatornetze 23, 64, 64a kann mit Hilfe der annähernd vollkommen abgeschlossenen Räume 72 ein arbeitserleichterndes sukzessives Entleeren des Innenraumes des zylindrischen Mantels erreicht werden.

An sich ist die Aufnahme zweier übereinandergelegener Katalysatorbetten in einem Reaktor aus der DE-A-3 022 815 bekannt. Insofern sollen die darauf gerichteten und im Zusammenhang damit genannten Merkmale nur in Verbindung mit weiteren Merkmalen der Erfindung Gültigkeit haben.

Einem zylindrischen Mantel ist als äquivalent ein annähernd zylindrischer Mantel gleichzusetzen.

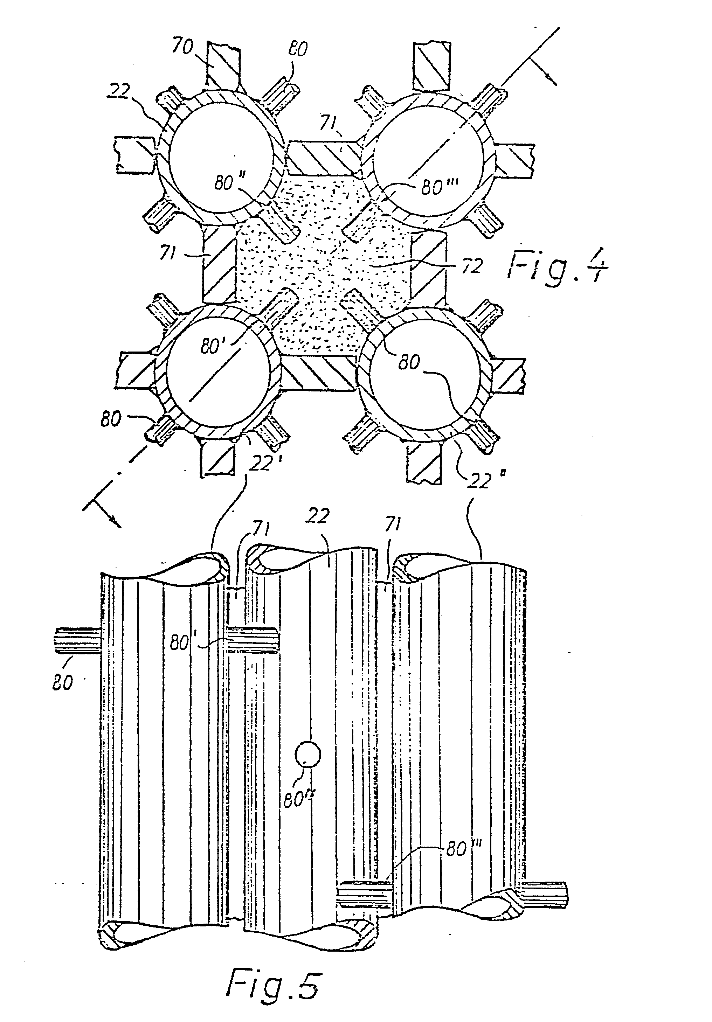

Die Rohre 22 weisen gemäss Fig. 4 und Fig. 5 winkelmässig gleichartig am Umfang verteilte, radial auswärts gerichtete Stifte 80 auf, die in den Strömungsraum 72 und das Katalysatorbett ragen und, wie Fig. 1 erkennen lässt, in Längsrichtung der Rohre sternförmig an diesen angeordnet sind. Die Stifte, die in den Strömungsraum 72 ragen, sind in unterschiedlicher Höhe angeordnet; so ist der Stift 80′ in Fig. 5 oberhalb des Stiftes 80″ und dieser wiederum oberhalb des Stiftes 80‴ angeordnet.

Die Länge der Stifte 80 beträgt etwa ¼ – ⅓ des Abstandes zwischen zwei diagonal benachbarten Rohren, so z. B. der Rohre 22′ und 22″ in Fig. 2.

Die Stifte sind vorzugsweise auf die Rohre aufgeschossen.

Sie können auch als im Querschnitt quadratische Stifte wie auch mit einem Kopf in Form eines Nietes ausgebildet sein.

## Patentansprüche

1. Stehender Reaktor zur Herstellung von Methanol, mit einem zylindrischen Gehäuse mit das Katalysatorbett im Inneren des Gehäuses durchsetzenden, parallel zur Reaktorlängsachse verlaufenden Austauscherrohren, sowie einem das Katalysatorbett tragenden, gasdurchlässigen Boden oder Netz im unteren Reaktorabschnitt, bei dem das Gehäuse durch eine obere Haube abgeschlossen ist, das Katalysatorbett von einem zylindrischen Mantel innerhalb des Gehäuses umschlossen ist, der bei abgehobener Haube mit dem Katalysatorbett in Längsrichtung des Reaktors aus dessen Gehäuse heraushebbar ist, dadurch gekennzeichnet, dass der zylindrische Mantel aus Flossenrohren (12, 58) gebildet ist, dass in die oberen und unteren Abschnitte (14, 15 bzw. 62, 63) der Flossenrohre (12, 58) des zylindrischen Mantels die abgebogenen Enden (20, 21 bzw. 59, 60) von den Innenraum des zylindrischen Mantels gleichmässig durchsetzenden, parallel zur Reaktorlängsachse verlaufenden Rohren (22, 61) münden, und dass die den zylindrischen Mantel bildenden Flossenrohre (12, 58) im unteren Reaktorabschnitt in eine Ringleitung (16, 63a) münden, und der das Katalysatorbett tragende, gasdurchlässige Boden oder das Netz (23, 64a) auf Höhe dieser Leitung (16, 63a) an dieser oder kurz darüber an den Rohrabschnitten (15, 63a) des zylindrischen Mantels befestigt ist.

2. Reaktor nach Anspruch 1, dadurch gekennzeichnet, dass die den Innenraum des zylindrischen Mantels parallel zur Reaktorlängsachse durchsetzenden Rohre (22, 61) derart mit Flossen (70, 71) versehen sind, dass die Rohre und Flossen benachbarter Rohre in sich abgeschlossene, mit Katalysatormasse gefüllte Strömungsräume (72) bilden von regelmässigem, geometrischem Querschnitt bilden.

3. Reaktor nach Anspruch 1, dadurch gekennzeichnet, dass der Innenraum des zylindrischen Mantels zur Aufnahme zweier übereinander angeordneter Abschnitte des Katalysatorbettes unterteilt ist und die Gaszuführungsleitung (56) unterhalb des oberen und oberhalb des unteren Bettabschnittes in den Reaktor mündet.

4. Reaktor nach Anspruch 3, dadurch gekennzeichnet, dass sich oberhalb der Gaseintrittsleitung (56) in den Reaktor ein zweiter Katalysatorboden oder –netz (64) über den Querschnitt des zylindrischen Mantels erstreckt, der von den Austauscherrohren (61) durchsetzt ist.

5. Reaktor nach Anspruch 2, dadurch gekennzeichnet, dass die mit ihren Flossen (70, 71) in sich abgeschlossene, mit Katalysatormasse gefüllte Strömungsräume (72) von regelmässigem geometrischen Querschnitt bildenden Austauscherrohre (22) mit radialen, symmetrisch in die Strömungsräume ragenden Stiften (80) versehen sind, wobei die Stifte eines Strömungsraumes (72) in unterschiedlicher Höhe an ihren Rohren (22) angeordnet sind.

6. Reaktor nach Anspruch 5, dadurch gekennzeichnet, dass die Stifte (80) eine Länge aufweisen, die etwa ¼ – ⅓ des Abstandes zweier diagonal benachbarter Rohre (22′, 22″) ausmacht.

7. Reaktor nach Anspruch 5 und 6, dadurch gekennzeichnet, dass die Stifte (80) auf die Rohrwand aufgeschossen sind.

## Claims

1. Standing reactor for the production of methanol with a cylindrical casing having exchange pipes interspersed in the catalyst bed, parallel to the longitudinal axis of the reactor, and a gas-permeable base or net supporting the catalyst bed in the lower section of the reactor, the casing being enclosed by an upper cover, the catalyst bed being enclosed by a cylindrical mantle, which when the cover is removed can be lifted out of the casing of the reactor in a longitudinal direction together with the catalyst bed, characterised in that the cylindrical mantle is composed of finned pipes (12, 58) that pipes (22, 61) which are parallel to the longitudinal axis of the reactor and are regularly interspersed in the interior of the cylindrical mantle join the upper and lower sections (14, 15 and 62, 63) of the finned pipes (12, 58) of the cylindrical mantle, and that the finned pipes (12, 58) forming the cylindrical mantle emerge into a circular pipe (16, 63a) in the lower reactor section and the gas-permeable base or net (23, 64a) supporting the catalyst bed at the level of these pipes (16, 63a) is attached to these or slightly above them to pipe sections (15, 63a).

2. Reactor according to claim 1, characterised in that the pipes (22, 61) running through the interior of the cylindrical mantle parallel to the longitudinal axis of the reactor are provided with fins (70, 71) in such a way that the pipes and fins of adjacent pipes form closed flow chambers (72) of regular geometric cross section filled with the catalyst mass.

3. Reactor according to claim 1, characterised in that the interior of the cylindrical mantle is divided so as to receive two sections of the catalyst bed arranged one on top of the other and the gas inlet pipe (56) emerges into the reactor below the top and above the bottom sections of the bed.

4. Reactor according to claim 3 characterised in that a second catalyst bed base or net (64) extends across the cylindrical mantle above the gas inlet pipe (56) into the reactor and is interspersed with exchange pipes (61).

5. Reactor according to claim 2 characterised in that the exchange pipes (22), which with their fins (70, 71) form closed flow chambers (72) of a regular geometric cross section filled with the catalyst mass, are provided with pins (80) which project radially and symmetrically into the flow chambers, the pins of flow chamber (72) being arranged at different heights on their pipes (22).

6. Reactor according to claim 5 characterised in that the pins (80) are of a length of approximately ¼ – ⅓ of the distance between two diagonally adjacent pipes (22′, 22″).

7. Reactor according to claims 5 and 6 characterised in that the pins (80) are shot onto the pipe wall.

**Revendications**

1. Réacteur placé debout pour la fabrication de méthanol, avec un carter cylindrique, avec des tubes échangeurs s'étendant parallèlement à l'axe longitudinal du réacteur et traversant le lit de catalyseur à l'intérieur du carter, ainsi qu'avec un fond perméable aux gaz ou bien un treillis, portant le lit de catalyseur, dans la partie inférieure du réacteur, réacteur dans lequel le carter est fermé par un capot supérieur, le lit de catalyseur étant entouré d'une enveloppe cylindrique à l'intérieur du carter, qui, lorsque le capot est enlevé, peut être soulevée avec le lit de catalyseur hors du carter du réacteur, en direction longitudinale de celui-ci, réacteur caractérisé en ce que l'enveloppe cylindrique est constituée par des tubes à ailerons (12, 58), tandis que dans les parties supérieure et inférieure (14, 15) ou bien (62, 63) des tubes à ailerons (12, 58) de l'enveloppe cylindrique, débouchent les extrémités recourbées (20, 21 ou bien 59, 60) de tubes (22, 61) traversant uniformément l'espace interne de l'enveloppe cylindrique et s'étendant parallèlement à l'axe longitudinal du réacteur, tandis que les tubes à ailerons (12, 58) constituant l'enveloppe cylindrique, débouchent dans la partie inférieure du réacteur dans une canalisation annulaire (16, 63a), et le fond perméable aux gaz ou bien le treillis (23, 64a) portant le lit de catalyseur, étant fixé au niveau de cette canalisation (16, 63a) ou bien peu au-dessus, aux parties inférieures (15, 63) des tubes à ailerons de l'enveloppe cylindrique.

2. Réacteur selon la revendication 1, caractérisé en ce que les tubes (22, 61) traversant parallèlement l'axe longitudinal du réacteur l'espace interne de l'enveloppe cylindrique, sont munis d'ailerons (70, 71) de façon que les tubes et les ailerons du tube voisin constituent des chambres d'écoulement (72) fermées en soi, et de sections géométriques régulières, qui sont remplies par la masse du catalyseur.

3. Réacteur selon la revendication 1, caractérisé en ce que l'espace interne de l'enveloppe cylindrique est subdivisé pour recevoir deux parties disposées l'une au-dessus de l'autre du lit de catalyseur, et la canalisation d'alimentation en gaz (56) débouche dans le réacteur au-dessous de la partie supérieure du lit et au-dessus de la partie inférieure du lit.

4. Réacteur selon la revendication 3, caractérisé en ce que , au-dessus de la canalisation d'alimentation en gaz (56), un second fond de catalyseur ou un second treillis de catalyseur (64) s'étend dans le réacteur sur toute la section transversale de l'enveloppe cylindrique, et est traversé par les tubes échangeurs (61).

5. Réacteur selon la revendication 2, caractérisé en ce que les tubes échangeurs (22) constituant avec leurs ailerons (70, 71) des chambres d'écoulement (72), fermées en soi, remplies par la masse du catalyseur et de sections géométriques régulières, sont munis de goujons (80) faisant saillie symétriquement dans les chambres d'écoulement, les goujons d'une chambre d'écoulement (72) étant disposés à des niveaux différents sur leurs tubes (22).

6. Réacteur selon la revendication 5, caractérisé en ce que les goujons (80) ont une longueur qui se situe à peu près entre un quart et un tiers de la distance de deux tubes diagonalement voisins (22′, 22″).

7. Réacteur selon les revendications 5 et 6, caractérisé en ce que les goujons (80) sont implantés au pistolet sur la paroi du tube.

Fig.1    Fig.2

0155341

# Fig. 3

Fig.4

Fig.5